Europäisches Patentamt

European Patent Office (11) Publication number: **0 003 589**

Office européen des brevets **B1**

(12) **EUROPEAN PATENT SPECIFICATION**

(45) Date of publication of patent specification: **11.04.84**  (51) Int. Cl.³: **A 61 K 9/20**

(21) Application number: **79100337.9**

(22) Date of filing: **05.02.79**

(54) Antacid tablet formulation.

(30) Priority: **06.02.78 GB 466978**

(43) Date of publication of application:
**22.08.79 Bulletin 79/17**

(45) Publication of the grant of the patent:
**11.04.84 Bulletin 84/15**

(84) Designated Contracting States:
**BE CH DE FR GB LU NL SE**

(56) References cited:
**FR - A - 2 007 475
FR - A - 2 230 341
FR - A - 2 260 993
FR - A - 2 300 550
GB - A - 1 057 940
US - A - 2 550 489
US - A - 2 581 035
US - A - 2 912 358**

(73) Proprietor: **THE WELLCOME FOUNDATION
LIMITED
183-193 Euston Road
London NW1 2BP (GB)**

(72) Inventor: **Harden, David
25 Sandhurst Road
Sidcup, Kent (GB)**
Inventor: **Gayst, Stephen
19 Arthur Street
Double Bay New South Wales (AU)**

(74) Representative: **Berg, Wilhelm, Dr. et al,
Dr. Berg, Dipl.-Ing. Stapf, Dipl.-Ing. Schwabe, Dr.
Dr. Sandmair Patentanwälte Postfach 860245
D-8000 München 86 (DE)**

Courier Press, Leamington Spa, England.

## Antacid tablet formulation

The present invention relates to tablets which rapidly disperse in solution and are useful for the treatment of gastric hyperacidity. The present invention also provides methods for preparing such tablets.

Formulations used to combat gastric hyperacidity conventionally contain an insoluble base or an alkaline salt with a weak acid of magnesium or aluminium or a mixture of such bases or salts. Occasionally insoluble bases or alkaline salts with weak acids of sodium, calcium and bismuth are also included in the formulations together with the said magnesium and/or aluminium bases or salts. Thus typical formulations often contain aluminium hydroxide, magnesium hydroxide or magnesium carbonate. These antacid formulations act by neutralising excess acidity in the stomach. To give rapid relief of gastric hyperacidity it is necessary that antacid formulations give rapid release of the active ingredients on administration.

Unfortunately, the formulations at present marketed are often deficient in this respect. Antacid formulations currently available are normally in tablet or suspension form and of these the suspension form gives the most rapid release of active ingredients. A new form of antacid tablet has now been developed which not only gives a far more rapid release of ingredient than those antacid tablets which have gone before but also gives a more rapid release of ingredients than some antacid suspensions presently available. Such tablets contain a disintegrating agent that has a capacity to swell substantially in the presence of water.

Accordingly, the present invention provides a dispersible tablet for the treatment of gastric hyperacidity, which comprises 50—90% of a conventional antacid component and 5—15% of a disintegrating agent having a swelling capacity of between 5 and 100 ml/g.

All percentages herein are expressed as weight/weight unless otherwise stated.

Antacid components suitable for inclusion in the tablets of the present invention are, for example, pharmaceutically acceptable metal bases or alkaline salts with weak acids conventionally used in antacid formulations (see, for example, Martindale, the Extra Pharmacopoeia, 27th Ed., for a list of conventional antacid formulations and their ingredients), which have a solubility in water of less than 1 part in 70 under neutral conditions.

GB—A—1,269,987 (=FR—A—2,007,475) discloses inter alia, solid antacid formulations which are claimed to revert to suspensions on contact with water. However the only disclosure of a compressed tablet formulation provides products with unacceptable physical properties and, in as far as could be determined, would not be a dispersible tablet.

FR—A—2,300,550, 2,260,993 and 2,230,341 disclose tablets in which the ingredient is present in high concentration and in which a disintegrating agent having a swelling capacity of greater than 5 mg/g. However the disclosures are concerned with the provision of tablets having good disintegration times (eg less than 15 minutes) and are not dispersible tablets nor would they exhibit the properties of a dispersible tablet.

It is usual to include a mixture of insoluble metal compounds in the tablets of the present invention but the invention also includes the case where only one such compound is included. The tablets contain 50 to 90%, preferably 60 to 70% of the antacid component(s).

Suitable insoluble metal compounds include those of calcium, such as calcium carbonate, of bismuth, such as bismuth carbonate and dismuth subnitrate, of aluminium, such as aluminium hydroxide, aluminium oxide, aluminium phosphate and aluminium glycinate, and of magnesium, such as magnesium hydroxide, magnesium carbonate and magnesium silicate. Mixed bases and/or alkaline salts, that is to say bases and/or salts formed from more than one metal such as sodium polyhydroxy-aluminium monocarbonate, sodium magnesium aluminium silicate, magnesium aluminium hydroxide and magnesium aluminate, are also suitable.

Particularly suitable insoluble metal compounds are those of aluminium and magnesium. Thus, aluminium hydroxide is preferred compound for inclusion in the tablets of the present invention. Preferred magnesium compounds include magnesium carbonate and, in particular, magnesium hydroxide. It has been found that a combination of aluminium hydroxide and magnesium hydroxide provides a particularly favourable antacid component for the tablets of the present invention. Suitably the aluminium hydroxide and the magnesium hydroxide are in weight ratio of from 4:1 to 1:4 and preferably in a weight ratio of from 4:1 to 1:2. Tablets containing these ingredients may suitably contain from 200 to 1000 mg of these compounds per tablet, for example 400 to 500 mg per tablet. A suitable dose for the treatment of gastric hyperacidity in man is 1 to 4 tablets repeated as required.

As used herein, the swelling capacity of a disintegrating agent is defined as the volume (ml) to which 1 g. of a test tablet containing 95% of the dry, disintegrating agent and 5% of polyvinylpyrollidone (K30) will swell when in contact with an excess of water at a temperature of 21°C. It is determined by granulating the disintegrating agent (2 g) with 10% polyvinylpyrollidone (K30) (1 ml) and drying the resultant granules at 60°C. Compression of the granules to a hardness value of 12 kg. provides test tablets having a diameter of 15 mm and a weight of approximately 900 mg. Each tablet is then accurately weighed and placed on the bottom of a 25 ml. measuring cylinder. A nylon disc of 8 mm thickness and having two grooves provides a close, but sliding, fit in the measuring cylinder, resting on the top of the tablet. The grooves are disposed opposite each other on the circumference of the disc in a direction at right angles to the plane thereof and allow for a thin hypodermic needle to be inserted between the disc and the glass wall of the measuring cylinder. A 5 g. weight is placed on the nylon disc and water injected through one of the grooves into the space surrounding the tablet; the other groove allowing for air to be displaced. When the water level is above the top of the disc, the needle can be removed and water added until it is in excess, e.g. 25 ml. The volume under the disc is then noted at periodic intervals until there is no further increase in absorption. In some cases, disintegrating agents absorb water to form viscous gels, and this slows down the rate of absorption necessitating a longer interval, such as 48 hours, before maximum swelling is achieved.

On completion of swelling, the final volume is read and corrected to the corresponding value for 1 g. of the tablet, i.e. the value for the swelling capacity. The whole operation should preferably be performed at an approximately constant room temperature, for example 21°C.

Disintegrating agents which have a swelling capacity greater than 5 ml/g. and less than 100 ml/g. and which therefore may be used in the present invention include calcium carboxy methyl celluloses, such as E.C.G. 505, low viscosity sodium carboxy methyl celluloses, such as Copagel and Nymcel, guar based vegetable gums, such as Supercol U and Supercol NG-1, a sodium alginate, such as alginate YZ, cross-linked sodium carboxymethyl-celluloses, such as CLD, cross-linked polyvinyl-pyrrolidones, such as Plasdone XL, low substituted hydroxypropylcelluloses, such as L-HPC, cation exchange resins, such as amberlite IRP-88, and sodium starch glycolates, such as Primojel or Explotab (Trade Marks). The most preferred disintegrating agents are Primojel and Explotab. The disintegrating agents will normally have a swelling capacity of less than 60 ml/g.

The percentage of disintegrating agent which is incorporated in the tablets of the invention will depend to a large extent on the swelling capacity of the disintegrating agent. Thus, if the disintegrating agent is one which has a high swelling capacity, such as Primojel or Explotab, the amount of disintegrating agent present will normally be between 5 and 10%. However, if a disintegrating agent of low swelling capacity is used then it will normally be present in a greater amount, i.e. between 8 and 15%.

The particle size, as hereinafter defined, of the antacid component will normally be below 100 μm and preferably below 50 μm. As used herein, the particle size of the antacid component is defined in terms of the "weight median diameter" hereinafter referred to as W.M.D. Thus, each particle is considered as a sphere having a volume identical with the actual particle and the W.M.D. is that "diameter", wherein 50% of these hypothetical spheres have a larger diameter than that figure and 50% a smaller diameter than that figure. The W.M.D. may be determined using a Coulter counter in which the antacid component dispersed in an electrolyte comprising an aqueous solution of, for example, sodium chloride, saturated with the antacid component is passed through a small orifice in a tube on either side of which is immersed an electrode. The changes in resistance as particles pass through the orifice generate voltage pulses whose amplitudes are proportional to the volume of the particles. The pulses are amplified, and the numbers counted at different threshold levels. From this data the distribution of the suspended particles and hence the W.M.D. may be determined.

The particle size of the antacid component may readily be reduced, if desired, by precipitation techniques or by grinding the particles with any apparatus or by any other method known in the art suitable for such purposes. In particular, the hammer mill, which can be used with either the rigid or the swing hammer type and is conveniently combined with a fan and a cyclone for collecting the material, is preferred.

In order to reduce flatulence anti-foam agents, such as siloxanes for example polymethyl-siloxane and polydimethylsiloxane, are often included in conventional antacid formulations. Thus, in one preferred embodiment the tablets of the present invention will also contain an anti-foaming agent. Suitably the tablet will contain between 1 and 5% of an anti-foaming agent. It has been found that certain antacid materials and particularly aluminium hydroxide inhibit the anti-foaming properties of polydimethylsiloxane (see *J. Pharm. Sci*, *55*, 538, 1966) and when such an antacid material is incorporated into the tablets of the invention it has been found to be convenient to separate it from the anti-foaming agent. It has been found that compression coating the anti-foaming agent with one of the other ingredients of the tablets, for example magnesium hydroxide in the case where both aluminium and magnesium hydroxides are used as the antacid component, to give a central core around which the aluminium hydroxide or other antacid material may be formulated gives a satisfactory separated product. Alternatively, a mixed granule system compressed as a conventional tablet gives satisfactory separation of the anti-foam agent.

Other components which may be incorporated in the tablets of the present invention include granulating agents, lubricating agents and binder/disintegrants and pharmaceutical excipients conventionally used in tablet formulations. Granulating agents will normally be included in the tablets and suitable granulating agents include starch in the form of mucilage, starch derivatives, such as starch "Snow Flake", cellulose derivatives, such as methylcellulose, gelatin and polyvinylpyrollidone. Polyvinylpyrollidone is particularly preferred. Lubricating agents are added to prevent the tablets from adhering to the punches and dies of the automatic tabletting equipment. Magnesium Stearate, Magnesium Lauryl Sulphate, and Sodium Lauryl Sulphate are convenient lubricating agents for inclusion in the tablets. Binder-disintegrants, as the name implies, serve a dual purpose in holding the tablets together until they come into contact with aqueous media when they aid the tablets' disintegration. Avicel, a microcrystalline cellulose, and L-HPC are particularly suitable examples of such binder/disintegrants. Conventional pharmaceutical excipients such as dyes, flavourings, surfactants, preservatives and the like may be included. It has been found that the inclusion of mannitol and/or lactose in the tablets of the present invention results in particularly advantageous products.

In another aspect of the present invention, there is provided a method of preparing a tablet as hereinbefore defined, which comprises the compression on standard machinery of a formulation containing 50—90% of a conventional antacid component, as hereinbefore defined and 5-15% of the disintegrating agent having a swelling capacity of between 5 and 100 ml/g.

In order to aid the compression of the formulation into a tablet the formulation will normally be granulated before it is compressed. This granulation step comprises for example, mixing the antacid component with the disintegrating agent in a dry state at slow speed, for example around 15 rev/min in a planetary mixer, followed by wet mixing for up to about 30 minutes with a granulating solution, together with additional solvent, if necessary, for maintaining the consistency of the mass. The material can then be milled and either tray-dried or dried in a fluidised bed. The dry granulated material is then sifted and a lubricant added. The granules are then compressed on standard machinery to the specified hardness in the conventional manner.

Whilst water may be used as the solvent in the granulating solution this may lead to a reduction in the dispersion properties of the tablets. We have found that the combination of water with a water miscible organic solvent, such as alcohol, provides an acceptable solvent for the granulating solution.

When the antacid component has good flow properties there may be no need to include a granulation step in which case the ingredients will be mixed together, a lubricant added and the resulting material compressed into tablets.

The tablets of the present invention may either be administered in tablet form or sucked or alternatively may be placed in water to give a finely dispersed suspension. All forms of administration give a very rapid onset of neutralising power together with a high neutralisation capacity. The formulations of the present invention have a radically different therapeutic profile and far superior properties from antacid preparations on the market.

The following Examples, which illustrate the invention, should in no way be construed as a limitation thereof.

### Example 1

| Granule: | Aluminium Hydroxide | 230 g |
|---|---|---|
| | Magnesium Hydroxide | 230 g |
| | Mannitol | 100 g |
| | Primojel | 50 g |
| | Avicel (internal) | 56 g |
| Blend: | Avicel (external | 66 g |
| | Magnesium Stearate | 7.3 g |

The granule contents were mixed together in conventional manner, for example by using Z-blade mixer. The mixed granule ingredients were wetted with a solution consisting of aqueous alcohol (alcohol, water content 50:50) sifted and passed through a 1400 $\mu$ sieve and the granules dried on a fluid bed drier until the outlet temperature was 46°C. The dried granules were passed through a 1000 $\mu$ sieve.

The avicel (external) and magnesium stearate (sifted to 150 $\mu$) were blended with the dried granules and the mixture compressed to give tablets of hardness 7.9 Kg., which give a fine dispersion (dispersion time 35—40 secs.) in water at room temperature.

### Example 2

Tablets were prepared as described in Example 1 except that the Primojel was replaced by Explotab (50 g). The tablets had a hardness of 8.8 kg and gave a fine dispersion (dispersion time 30 secs.) in water at room temperature.

### Example 3

| Granule Ingredients: | Aluminium Hydroxide | 460 g |
|---|---|---|
| | Lactose | 200 g |
| | Primojel | 61 g |
| | Avicel pH 101 | 100 g |

The granule ingredients were granulated by the method described in Example 1. The dried granules were blended with magnesium stearate (8.2 g) and the mixture pressed into tablets. The tablets had a hardness of 11.2 Kg and gave a fine dispersion (dispersion time 10 secs.) in water at room temperature.

### Example 4

| Tablets of Aluminium hydroxide | 200 mg |
|---|---|
| Magnesium hydroxide | 250 mg |
| Simethicone N.F. | 20 mg |

#### Part I

| Aluminium hydroxide | 200 g |
|---|---|
| Magnesium hydroxide | 200 g |
| Mannitol | 87 g |
| Primojel | 43 g |
| Avicel pH 101 | 50 g |

#### Part II

| Magnesium hydroxide | 50 g |
|---|---|
| Tricalcium phosphate | 25 g |
| Avicel pH 101 | 15 g |
| Primojel | 9 g |
| Simethicone N.F. | 20 g |

#### Part III

| Avicel | 68 g |
|---|---|
| Lubricant | 7 mg |

Part I and II are granulated separately as described in Example I, the simethicone being incorporated in the granulating solvent for Part II. The granules were dried and sifted by the procedure of Example 1. The two granulations mixed together, Part III added and tablets were compressed to give a hardness of 8 Kg and a dispersion time of 32 seconds at room temperature.

## Claims

1. A tablet comprising a conventional antacid component as herein defined characterised in that the tablet is dispersible, the antacid component is present in an amount of from 50 to 90%, by weight, and the tablet additionally comprising from 5 to 15% by weight of a disintegrating agent having a swelling capacity of between 5 and 100 ml/g.

2. A dispersible tablet as claimed in claim 1 characterised in that the antacid component comprises a pharmaceutically acceptable metal base or alkaline salt thereof with a weak acid.

3. A dispersible tablet as claimed in either claim 1 or claim 2 characterised in that the antacid component has a solubility in water of less than 1 part by weight in 70.

4. A dispersible tablet as claimed in any one of claims 1 to 3 characterised in that the antacid component is a compound of calcium, bismuth, aluminium or magnesium.

5. A dispersible tablet as claimed in any one of claims 1 to 4 characterised in that the antacid component is a compound of calcium or magnesium.

6. A dispersible tablet as claimed in any one of claims 1 to 5 characterised in that the antacid component is selected from one or more of aluminium hydroxide magnesium hydroxide, magnesium carbonate and magnesium silicat.

7. A dispersible tablet as claimed in any one of claims 1 to 6 characterised in that the antacid component comprises aluminium hydroxide and magnesium hydroxide in a weight ratio of 4:1 to 1:4.

8. A dispersible tablet as claimed in any one of claims 1 to 7 characterised in that the antacid component has a weight median diameter, as herein defined, of less than 100 $\mu$m.

9. A dispersible tablet as claimed in claim 8 characterised in that the weight median diameter is less than 50 $\mu$m.

10. A dispersible tablet as claimed in any one of claims 1 to 9 characterised in that the disintegrating agent is selected from calcium carboxymethyl celluloses, low viscosity sodium carboxymethyl celluloses, guar based vegetable gums, a sodium alginate, cross-linked sodium carboxymethyl celluloses, cross-linked poly-vinylpyrrolidones, low substituted hydroxy-propyl celluloses, cation exchange resins and sodium starch glycolates.

11. A dispersible tablet as claimed in any one of claims 1 to 10 characterised in that the disintegrating agent is a sodium starch glycolate.

12. A dispersible tablet as claimed in any one of Claims 1 to 11 characterised in that the disintegrating agent has a swelling capacity of less than 60 ml/g.

13. A dispersible tablet as claimed in any one of claims 1 to 11 characterised by comprising from 5 to 10% of disintegrating agent.

## Revendications

1. Comprimé comprenant un composant antiacide classique tel que défini ci-dessus, caractérisé en ce que le comprimé est dispersable, le composant antiacide est présent en une quantité de 50 à 90% en poids et le comprimé comprend, en outre, 5 à 15% en poids d'un agent de désintégration ayant une capacité de gonflement située entre 5 et 100 ml/g.

2. Comprimé dispersable suivant la revendication 1, caractérisé en ce que le composant antiacide comprend une base métallique pharmaceutiquement acceptable ou un sel alcalin de celle-ci issu d'un acide faible.

3. Comprimé dispersable suivant la revendication 1 ou 2, caractérisé en ce que le composant antiacide a une solubilité dans l'eau inférieure à 1 partie en poids dans 70.

4. Comprimé dispersable suivant l'une quelconque des revendications 1 à 3, caractérisé en ce que le composé antiacide est un composé du calcium, du bismuth, de l'aluminium ou du magnésium.

5. Comprimé dispersable suivant l'une quelconque des revendications 1 à 4, caractérisé en ce que le composant antiacide est un composé du calcium ou du magnésium.

6. Comprimé dispersable suivant l'une quelconque des revendications 1 à 5, caractérisé en ce que le composant antiacide comprend un ou plusieurs composés choisis entre l'hydroxyde d'aluminium, l'hydroxyde de magnésium, le carbonate de magnésium et le silicate de magnésium.

7. Comprimé dispersable suivant l'une quelconque des revendications 1 à 6, caractérisé en ce que le composant antiacide comprend de l'hydroxyde d'aluminium et de l'hydroxyde de magnésium dans un rapport pondéral de 4:1 à 1:4.

8. Comprimé dispersable suivant l'une quelconque des revendications 1 à 7, caractérisé en ce que le composant antiacide à un diamètre médian en poids, tel que défini ci-dessus, inférieur à 100 $\mu$m.

9. Comprimé dispersable suivant la revendication 8, caractérisé en ce que le diamètre médian en poids est inférieur à 50 $\mu$m.

10. Comprimé dispersable suivant l'une quelconque des revendications 1 à 9, caractérisé en ce que l'agent de désintégration est choisi parmi les carboxyméthylcelluloses calciques, les carboxyméthylcelluloses sodiques à basse viscosité, les gommes végétales à base de cyamopsis, un alginate de sodium, les carboxyméthylcelluloses sodiques réticulées, les polyvinylpyrrolidones réticulées, les hydroxypropylcelluloses à faible substitution, les résines échangeuses de cations et les amidons glycolates de sodium.

11. Comprimé dispersable suivant l'une quelconque des revendications 1 à 10, caractérisé en ce que l'agent de désintégration est un amidon glycolate de sodium.

12. Comprimé dispersable suivant l'une quelconque des revendications 1 à 11, caractérisé en ce que l'agent de désintégration a une capacité de gonflement de moins de 60 ml/g.

13. Comprimé dispersable suivant l'une quelconque des revendications 1 à 11, caractérisé en ce qu'il comprend 5 à 10% d'agent de désintégration.

## Patentansprüche

1. Tablette, die einen herkömmlichen antaciden Bestandteil gemäß hierin befindlicher Definition enthält, dadurch gekennzeichnet, daß die Tablette dispersibel ist, der antacide Bestandteil in einer Menge von 50 bis 90 Gew.-% vorhanden ist, und die Tablette zusätzlich 5 bis 15 Gew.-% eines Zersetzungsmittels mit einer Schwellkapazität von zwischen 5 und 100 ml/g enthält.

2. Dispersible Tablette nach Anspruch 1, dadurch gekennzeichnet, daß der antacide Bestandteil eine pharmazeutisch verträgliche Metallbase oder ein Alkalisalz davon mit einer schwachen Säure umfaßt.

3. Dispersible Tablette nach Anspruch 1 oder Anspruch 2, dadurch gekennzeichnet, daß der antacide Bestandteil eine Löslichkeit in Wasser von weniger als 1 Gew.-Teil in 70 aufweist.

4. Dispersible Tablette nach einem der Ansprüche 1 bis 3, dadurch gekennzeichnet, daß der antacide Bestandteil eine Verbindung von Calcium, Wismut, Aluminium oder Magnesium ist.

5. Dispersible Tablette nach einem der Ansprüche 1 bis 4 dadurch gekennzeichnet, daß der antacide Bestandteil eine Verbindung von Calcium, oder Magnesium ist.

6. Dispersible Tablette nach einem der Ansprüche 1 bis 5 dadurch gekennzeichnet, daß der antacide Bestandteil under einer oder mehreren der Verbindungen Aluminiumhydroxid, Magnesiumhydroxid, Magnesiumcarbonat und Magnesiumsilikat ausgewählt ist.

7. Dispersible Tablette nach einem der Ansprüche 1 bis 6, dadurch gekennzeichnet, daß der antacide Bestandteil Aluminiumhydroxid und Magnesiumhydroxid in einem Gewichtsverhältnis von 4:1 bis 1:4 enthält.

8. Dispersible Tablette nach einem der Ansprüche 1 bis 7, dadurch gekennzeichnet, daß der antacide Bestandteil einen Gewichtsmitteldurchmesser (weight median diameter) gemäß der hierin befindlichen Definition von weniger als 100 $\mu$m besitzt.

9. Dispersible Tablette nach Anspruch 8, dadurch gekennzeichnet, daß der Gewichtsmitteldurchmesser weniger als 50 $\mu$m beträgt.

10. Dispersible Tablette nach einem der Ansprüche 1 bis 9 dadurch gekennzeichnet, daß das Zersetzungsmittel unter Calciumcarboxymethylcellulosen, Natriumcarboxymethylcellulosen niedriger Viskosität, pflanzlichen Kautschuken auf Guar-Basis, einem Natriumalginat, vernetzten Natriumcarboxymethylcellulosen, vernetzten Polyvinylpyrrolidonen, niedrig substituierten Hydroxypropylcellulosen, Kationenaustauscherharzen und Natrium-Stärkeglykolaten ausgewählt ist.

11. Dispersible Tablette nach einem der Ansprüche 1 bis 10, dadurch gekennzeichnet, daß das Zersetzungsmittel ein Natrium-Stärkeglykolat ist.

12. Dispersible Tablette nach einem der Ansprüche 1 bis 11, dadurch gekennzeichnet, daß das Zersetzungsmittel eine Schwellkapazität von weniger als 60 ml/g besitzt.

13. Dispersible Tablette nach einem der Ansprüche 1 bis 11, dadurch gekennzeichnet, daß sie 5 bis 10% Zersetzungsmittel enthält.